# EUROPEAN PATENT APPLICATION

(11) **EP 3 885 820 A1**
(43) Date of publication of application: **29.09.2021**
(21) Application number: 21163026.4
(22) Date of filing: 17.03.2021
(51) Int. Cl.: G02C 11/00, G02C 7/16

(54) **DEVICE FOR LIMITING VISUAL IMPRESSIONS**

(30) Priority: 24.03.2020 SE 2050321
(71) Applicant: AMBICARE AB, 126 28 Hägersten (SE)
(72) Inventor: Widö, Thorunn, 126 28 Hägersten (SE)
(74) Representative: Ehrner & Delmar Patentbyrå AB

(57) **Abstract**

The present invention relates to a device (10) for limiting visual impressions, comprising a carrier structure (12) for arrangement around an eye area (EA) of a user, the carrier structure (12) comprising a first limiter (18) comprising first surface areas (20, 22) capable of limiting impressions incoming generally from above the eye area of the user, wherein the surface areas (20, 22) of the first limiter (18) are arranged to contact facial areas around the eye area of the user.

## Description

### TECHNICAL AREA

The present application relates to a device for limiting visual impressions and in particular visual impressions that may distract a person or cause stress, pain or the like symptoms to a person.

### BACKGROUND OF INVENTION

Many persons suffer from different problems that may be worsened by visual impressions. This may be persons for example having severe stress problems like severe fatigue syndrome, vicarious traumatization and compassion fatigue, suffer from migraine attacks, have sustained severe concussions like traumatic brain injury, TBI, or persons with neuropsychiatric functional disorders like autism or persons with concentration difficulties, like persons with attention deficit hyperactivity disorder, ADHD.

These persons are easily affected in different ways by visual impressions that in many cases worsen the conditions to a great extent. One way of handling this is to limit the visual impressions in different ways. For some persons, spectacles with tinted or coloured glasses that limit the light spectra of light incoming to the eyes may provide a remedy or at least a relief. For some persons, a limitation of the visual impression may be necessary. In view of this, some solutions have been developed that in some ways limit the field of view.

One example is therapeutic glasses for e.g. anxiety that are provided with opaque lenses in front of the eyes except for a horizontal slit in front of each eye. The slits are quite narrow, less than 10 mm, and extend from the inner edge of the spectacle frame to about the outer edge of the iris of a user. They are to be put on every time a user experiences symptoms of the condition for a time period until the symptoms are reduced.

Some drawbacks with these types of therapeutic glasses are for instance that they are not ideal to use throughout the day and in particular not to move around with due to the obscured field of view in particular downwardly in front of the user, such that the user cannot see his feet or the ground in front of him, which may cause nausea or dizziness. Moreover, any incoming light or other visual impressions from above are not completely removed by that solution, nor any incoming light or other visual impressions from the side. A further drawback is that the glasses are curved, i.e. the lenses follow the contour of the face leading to a wider field of view than with a straight lens. When the lens is curved, or follows the contour of the face, the opaque area has to be bigger to get the same effect, leading to less control and greater discomfort for the user.

Thus, there are improvements to be made in the field of limiting visual impressions for therapeutic treatment.

### BRIEF DESCRIPTION OF INVENTION

The aim of the present application is to remedy the drawbacks of the state of the art technologies. This aim is obtained by a device according to the features of the independent patent claim. Preferable embodiments form the subject of the dependent patent claims.

According to one aspect, a ddevice is provided for limiting visual impressions. The device may comprise a carrier structure for arrangement around an eye area of a user. Further, the carrier structure may comprise a first limiter comprising first surface areas capable of limiting impressions incoming generally from above the eye area of the user. The surface areas of the first limiter may be arranged to contact facial areas around the eye area of the user. The advantage with this solution is that visual impressions like light, movement and other sources originating from above the user are minimized, in order for the user to be adversely affected as little as possible.

According to a further feature of the invention, the carrier structure may further comprise a second limiter comprising second surface areas capable of limiting impressions incoming generally from an outer side of the eye area. Also here the surface areas of the second limiter may be arranged to contact facial areas around the eye area of the user. The second limiter then further minimizes visual impressions like light, movement and other sources, and in particular from the side.

The first and the second limiter then cooperate in the general limitation of visual disturbances.

As a further advantage of the present invention, at least regions of the surface areas in contact with the facial areas may be made of a flexible material, and in particular, the flexible material contacting the facial areas may be capable of providing a soft feeling to the user. This provides a limitation of the sensations that the user experiences, and for many users having conditions that are affected negatively by visual impressions, they might also be sensitive to facial contact, whereby a flexible and soft contact is positive.

In order to ascertain a high degree of limitation of visual impressions, the regions contacting the facial areas may be in abutment with the facial area, generally preventing all visual impressions incoming at those facial areas. This ensures that disturbances are limited as much as possible. As a further measure of limiting disturbances, inner surfaces of the surface areas may be arranged with a uniform colour.

According to a further aspect of the invention, it may further comprise a third limiter comprising third surface areas capable of limiting impressions in front of the eye area of the user. In this regard, the third surface areas of the third limiter may extend from generally each pupil of the user and outwards. Thus, the third limiter further limits disturbances of visual impressions to also include impressions coming at an outer angle in front of the user, and yet enabling the user to view and focus on objects straight in front of him or her. In order to further allow the user to view and focus on objects in front of him or her, the third surface area of the third limiter should be arranged to allow sight visibility in downwards directions. This may be especially important if the user is wearing the device while moving around because the third limiter does not limit the sight of view downwards in front of the user, so that he or she may view the hands and the feet as well as the ground in front of him or her.

Moreover, the third surface areas of the third limiter may be arranged as a part of a fourth surface area in front of the eye area and in that respect, the remainder of the fourth surface area may be transparent. With this solution, the device may be used in conjunction with spectacles and in this regard, the device may be an integral part of spectacles worn by a user or may be releasably attached to spectacles, for example as a so-called clip-on. Further, if spectacles are used, the remainder of the fourth surface area may comprise sight correcting features, enabling a user with eye correction needs to use the device according to the invention. Also, the remainder of the fourth surface area may comprise light spectra limiting features such as being tinted or having certain types of light filters.

According to a further aspect of the invention, the third, fourth and the fifth surface areas may be generally planar or only slightly curved and that the surface areas in front of each eye preferably are generally parallel to each other or only slightly inclined in relation to each other in relation to a horizontal line where the main object is that they should not follow the shape of the face. They might be slightly inclined towards the face in relation to a vertical line in order to reduce or minimize any visual obstruction from the device in a downward direction, for instance if the carrier structure is designed as a spectacle frame. Thus, the surface areas in front of the eyes of a user are not curved to follow the contour of the face of the user.

This has proven to be beneficial when using the device because the surface areas will not be placed so close to the eyes disturbing the user with eyelashes hitting the lenses. Further, it will limit the user's field of vision without the user feeling the lack of control, as opposed to when the surface is curved and the opaque area has to be bigger than necessary, still providing a good limiting function against visual impressions. Tests have shown that a majority of people that have used the device according to the application have experienced an improved ability to focus on tasks that they perform, due to the reduced disturbances, enabled by the limiters.

These and other aspects of, and advantages with, the present invention will become apparent from the following detailed description of the invention and from the accompanying drawings.

### BRIEF DESCRIPTION OF DRAWINGS

In the following detailed description of the invention, reference will be made to the accompanying drawings, of which
Fig. 1 shows a view of a face having defined eye areas,
Fig. 2 shows a perspective view of a first example of a device according to the invention,
Fig. 3 shows a front view of a carrier structure provided with surface areas having certain configurations,
Fig. 4 shows a front view of a carrier structure provided with surface areas having certain configurations,
Fig. 5 shows a front view of a carrier structure provided with surface areas having certain configurations,
Fig. 6 shows a perspective view of a second example of the device according to the invention, and
Fig. 7 shows a perspective view of a third example of the device according to the invention.

### DETAILED DESCRIPTION OF THE INVENTION

### Definitions

Carrier structure is meant to comprise any suitable structure that can carry visual impression limiters. Such carrier structures may be spectacles or glasses having spectacle frames that rest on the nose of a bearer and earpieces resting on the ears of a bearer. The carrier structure may further comprise structures that are designed to be attached to spectacles, such as so-called clip-ons.

Limiter is meant to any suitable component capable of limiting visual impressions from the surroundings that may be incoming to the eyes of a user of the device. The limiter may be an integral part of for instance spectacles or clip-ons or be components that are attached to these. The limiter is provided with surfaces that at least in some area are designed to limit visual impressions.

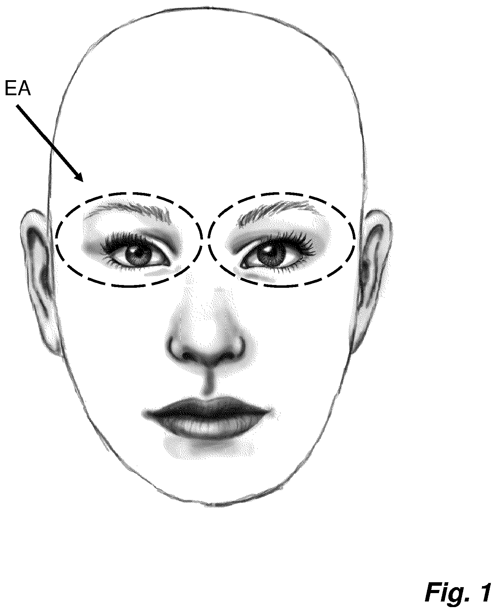

Eye area is meant to mainly include areas EA in the proximity of the eyes as shown in Fig. 1, and generally not the cheeks or higher regions of the brow.

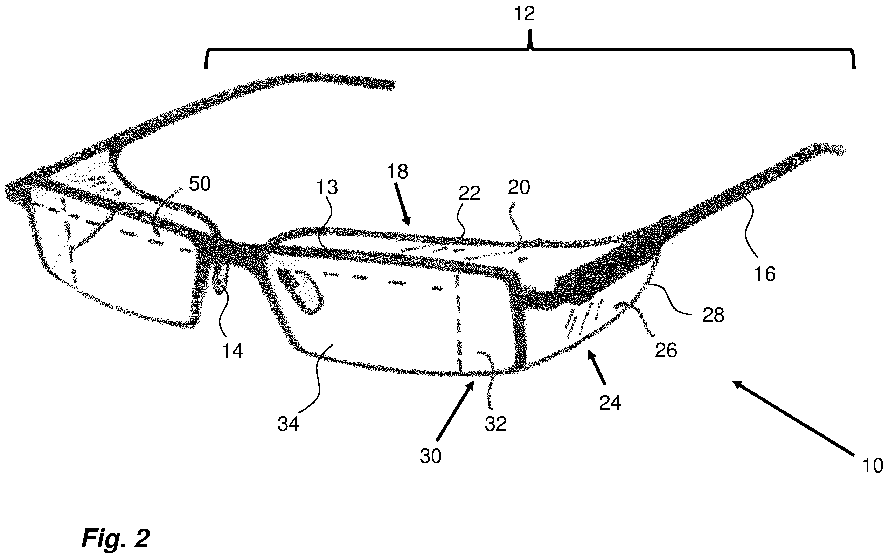

One non-limiting example of a device 10 according to the present application is shown in Fig. 2. The device 10 is arranged with a carrier structure 12 that in the example shown comprises a spectacle frame 13 having a support 14 for the nose of a user. The carrier structure 12 further comprises earpieces 16 for support on the ears of a user. On the carrier structure 12, a first limiter 18 is arranged to limit incoming light or other visual impressions from above the user. The first limiter 18 comprises first surface areas 20 that extend from an upper edge of the spectacle frame and towards an eye area of the user when worn. The first surface areas 20 may be an integral part of the spectacle frame 13, for instance molded together with the frame if the frame is manufactured from plastic. Alternatively, the first surface areas 20 may be attached to the frame 13, either permanently by for example gluing or melting, or releasably, for example by adhesive tape or by hook-and-loop fasteners. As an alternative, the frame and the first surface areas may be provided with attachment portions, which could comprise magnets and magnetically attractable material or tabs and recesses that are either discrete elements or extend along the contact surfaces between the frame and the first attachment portions.

According to one aspect, inner edges 22 of the first surfaces areas 20 are arranged to be in contact with an upper part of the eye area at least at regions thereof. This is to ensure that any incoming light and other visual impressions from above are limited as much as possible. Preferably the first surface areas 20 with the regions of the inner edges 22 extend at least from a vertical line coinciding with an outer edge of the eye to a vertical line coinciding with an inner edge of the eye. The first surface areas 20 may of course extend further. For example, the first surfaces areas 20 could extend along the whole breadth of the spectacle frame, i.e. also over a nose area of the user. The first surface areas 20 may also preferably extend from the frame 13 to at least a front part of the earpieces 16 in order to ascertain that no incoming light or other visual impressions from above may enter from the side of the eye area.

The first surface areas 20 may be made of a flexible material or have flexible properties such that the inner edges of the first surface areas provide a flexible contact with the upper part of the eye area. As an alternative, only the part of the first surfaces areas 20 closest to the eye area is flexible, either by using a different material closest to the eye areas or by having different properties of the material or design used for the first surface areas 20. In that regard, the thickness of the first surface areas 20 is reduced, either continuously or stepwise, in order to provide more flexible properties of the inner edges. In any case, the properties of the inner edge are preferably arranged to provide a soft sensation to a user. This is especially important for persons with certain conditions that are sensitive to facial contact in addition to being sensitive to visual impressions. This soft sensation may be obtained by providing the inner edge of the first surfaces areas with for example foam rubber, soft cloth or the like or by treating the material of the edges of the first surfaces such that they become very soft, which may be done with some plastic materials. As an alternative, a larger region of the first surface area, or the whole of the first surface are, may be made of a soft material, such as rubber, silicone, cloth, non-woven materials, leather, cork etc. The first surface areas 20 are preferably in abutment with the eye area in order to really ascertain that no light may "sneak" there between.

The device of the example of Fig. 2 further comprises a second limiter 24 arranged to the carrier structure 12. The second limiter 24 is arranged to limit incoming light or other visual impressions from the side of the eye area. The second limiter 24 comprises second surface areas 26 that extend from the earpieces 16 and downwards on the outer side of the eye areas. The second surface areas 26 may also preferably be arranged to the outer edges of the spectacle frame 13. The second limiter 24 may be designed and having the materials used in the same manner as with the first limiter 18. In this regard, it is naturally to be understood that for example the second limiter 24 may be removable and the first limiter 18 integral or vice versa or both be integral, or both be removable. The rear edges 28 of the surface areas 26 of the second limiter 24 may be in contact with a side part of the eye area at least at regions thereof, ensuring that any incoming light or other visual impressions from the side are limited as much as possible. As for the first limiter 18, the edges of the second limiter 24 in contact with the eye area of the user are preferably made of materials that provide a soft sensation to the user, for the same reasons as stated above. Moreover, the choice of material and/or design may be the same as for the first limiter. Further, the lower edges of the side surfaces of the second limiter may be provided with regions that are in contact with the eye areas, but may also be designed so as to leave a gap therebetween.

The example shown in Fig. 2 may further be provided with a third limiter 30. This third limiter 30 is arranged and designed with third surface areas 32 that limit incoming light or other visual impressions in the front of the user. The third surfaces areas 32 extend from the upper edge of the spectacle frame 13 and generally vertically downwards. Further, the third surface areas 32 may extend from an outer side edge of the spectacle frame 13, and/or from a front edge of the second surface areas 26 so that the second limiter 24 and the third limiter 30 provide a continuous limitation of incoming light or other visual impressions. The third surface areas 32 further extend inwards up to a point partly obscuring straight ahead. The third surfaces areas 32 may extend to a point coinciding with the centre of the pupil in a vertical direction or to a point coinciding with the outer edge of the iris in a vertical direction or the outer corner of the eye.

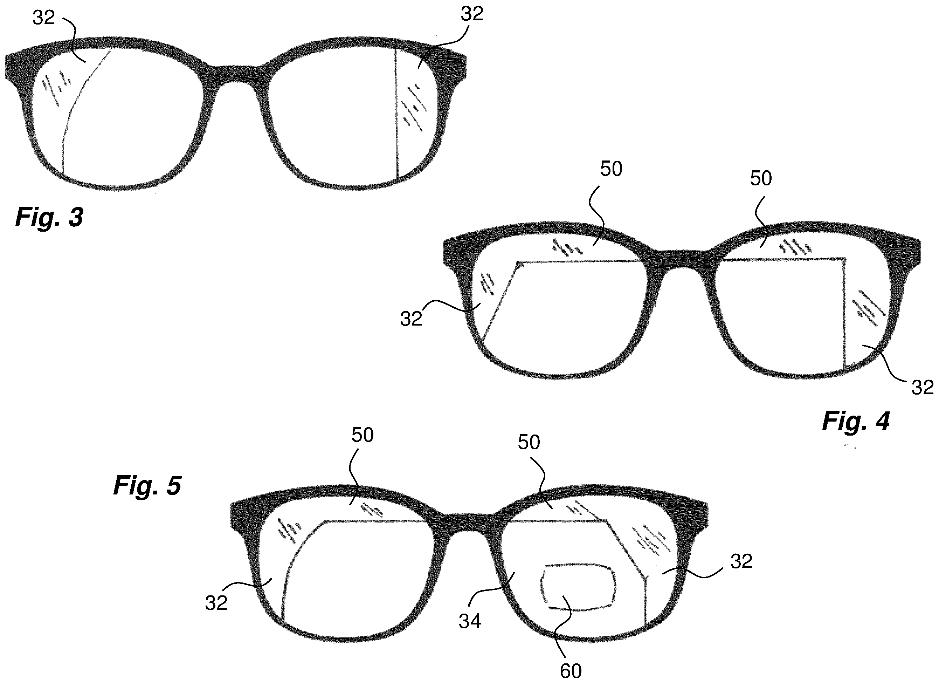

If a spectacle frame 13 is used as a carrier structure, it may be arranged with fourth surface areas 34, that may be in the form of lenses attached to and mounted in the spectacle frame 13. The lenses are preferably transparent and, in this regard, the third surface areas 32 of the third limiter 30 may form a part of the fourth surface areas 34. The third surface areas 32 may then be attached to the fourth surface areas 34 as an opaque film that may be fastened by an adhesive of by electrostatic adhering. It is also possible to provide the opacity in other ways such as grinding, blasting, painting or altering the manufacturing process when making the lenses. As an alternative, the third surface areas 32 may be an integral part of the fourth surface areas 34, i.e. the opaque third surface area 32 is formed when the lenses are manufactured. As a further alternative, the third surface areas 32 and the fourth surface 34 areas may be manufactured separately and joined to each other before mounting in the frame, or joined when both surface areas are mounted in the frame 13. In relation to the third surface areas, the boundaries may be generally vertical, as seen in Fig. 3, right side, or may be inclined upwards and inwards, either step-wise, Fig. 3, left side, along the entire boundary, Fig. 4, left side or along an upper part of the boundary, Fig. 5, right side, for example be generally vertical from the lower edge of the frame up to a certain point, after which the boundary may be inclined upwards and inwards. The inclination may for example start from a lower edge of the eye and upwards. The inclination may further not be straight but may be continuously curved, Fig. 5, left side. The third surface areas 32 may extend to the upper edge of the spectacle frame as seen in Fig. 3, right side, or extend to fifth surface areas 50 as seen in Fig. 4, right side, if such fifth surface areas 50 are provided.

A further aspect of the third, fourth and the fifth surface areas is that they are generally planar or only slightly curved and that the surface areas in front of each eye preferably are generally parallel to each other or only slightly inclined in relation to each other in relation to a horizontal line where the main object is that they should not follow the shape of the face. They might be slightly inclined towards the face in relation to a vertical line in order to reduce or minimize any visual obstruction from the device in a downward direction, for instance if the carrier structure is designed as a spectacle frame.

The fifth surface areas 50 may further be provided with the device 10. These surface areas 50 are designed to limit incoming light and other visual impressions coming from in front and above the user. The fifth surface areas 50 may on the one hand be a part of the carrier, structure, i.e. that for example the spectacle frame 13 is provided with a thicker upper part. As another example, the fifth surface areas 50 may be arranged in the same manner and with the same material as with the third surface areas 32.

The inner surfaces of all the surface areas may be arranged with uniform colour, that could be a dark colour. With this arrangement, visual disturbances may be further reduced in that the inner surfaces of the surface areas do not create any disturbances of their own. In this respect, the inner surfaces may also have matte structure in order to minimize possible light reflected on the surface areas.

The fourth surface areas 34 may further be provided with sight correcting features, 60. For example if a user has an eye defect such that he/she is unable to clearly view items or text at certain distances from the user, the lenses may be provided with the necessary correction features 60 such as being machined, e.g. ground and polished, or in other ways treated to provide the right eye-sight correction. For instance, if the lenses are manufactured by 3-D printing technology, the correcting features as well as both the third and the fifth surfaces areas may be made in the same process. Moreover, the fourth surface areas 34 may be provided with light spectra limiting features in order to remedy some conditions that the user may suffer from. These may for example be tinted in certain colours and/or may be provided with UV-filters.

The light spectra limiting features may either be arranged as films attached to the lenses or may be integrated in the material of the lenses of the fourth surface areas.

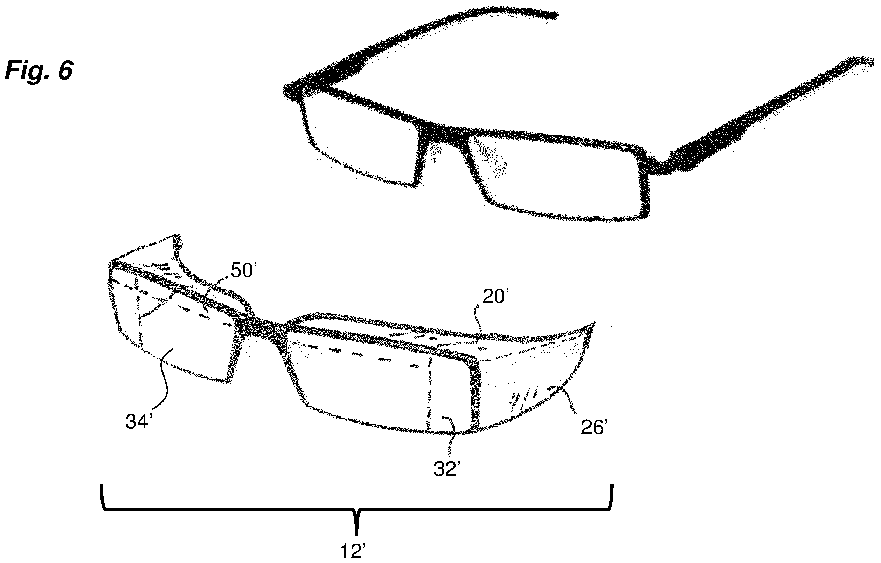

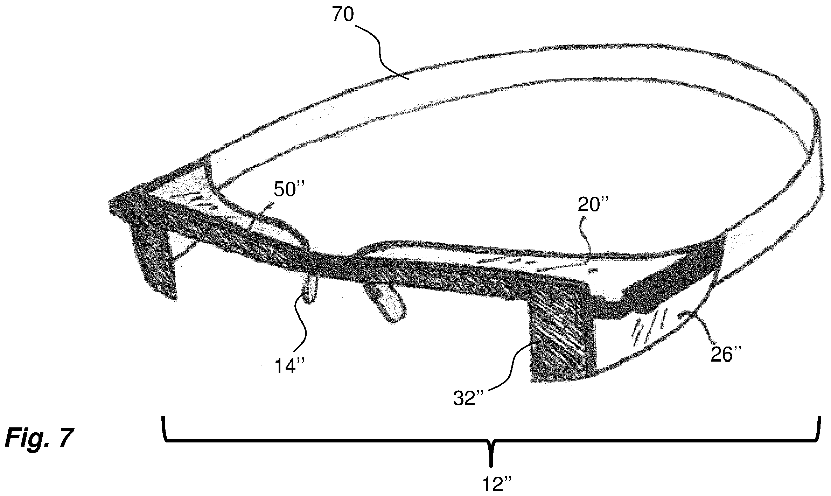

As an alternative to the above described device, it may also be designed as a device 10' of its own that may be attached to spectacles, as shown in Fig. 6, such as for example as clip-ons. The device 10' is then provided with its own carrier structure 12' and with the first 20', second 26', third 32', and fifth 50' surface areas. The alternative that is attached to spectacles may be designed as one unit as seen in Fig. 6 but may be designed as two discrete units, where each unit is arranged to be connected to each frame/lens part of the spectacles. Further, the device may be designed with so-called snap-in or click-in attachments that removably can attach the carrier structure to spectacles. Depending on the design, the device 10' may or may not be provided with the fourth surface areas 34'. As seen in Fig. 6, the carrier structure 12' is arranged with the fourth surface areas 34', which then may have the features as described above. As an alternative, the fourth surface 34 areas may be omitted, and its features be replaced by the lenses of spectacles to which the device 10' is to be attached. Such a solution not having the fourth surface areas is shown in Fig. 7. As is to be understood in this regard, the device 10" may also function completely without spectacles as shown in Fig. 7, if for example no eye correction is needed. Then the carrier structure 12" is a device of its own having user support 14" as well as first 20", second 26", third 32" and fifth 50" surface areas. A further alternative to earpieces is also shown in Fig. 7 where a band 70 or the like, for example an elastic band, may be used as support for the device 12".

Regarding the third, fourth and fifth surfaces areas, they should be arranged to allow sight visibility in downwards directions. This may be especially important if the user is wearing the device while moving around because the third limiter does not limit the sight of view downwards in front of the user, so that he or she may view the hands and the feet as well as the ground in front of him or her. Further, regarding manufacture of the device according to the invention, the whole device or parts of the device may be manufactured by using 3-D printing technology.

It is to be understood that the embodiment described above and shown in the drawings is to be regarded only as a non-limiting example of the invention and that it may be modified in many ways within the scope of the patent claims.

## Claims

1. Device (10) for limiting visual impressions, comprising a carrier structure (12) for arrangement around an eye area (EA) of a user, the carrier structure (12) comprising a first limiter (18) comprising first surface areas (20, 22) capable of limiting impressions incoming generally from above the eye area of the user, wherein the surface areas (20, 22) of the first limiter (18) are arranged to contact facial areas around the eye area of the user.

2. Device according to claim 1, wherein the carrier structure (12) further comprises a second limiter (24) comprising second surface areas (26, 28) capable of limiting impressions incoming generally from an outer side of the eye area,
wherein the surface areas (26, 28) of the second limiter (24) are arranged to contact facial areas around the eye area of the user.

3. Device according to any of the claims 1 to 2, wherein at least regions (22, 28) of the surface areas (20, 22) in contact with the facial areas are made of a flexible material.

4. Device according to any of the claims 3 to 4, wherein the regions (22, 28) contacting the facial areas are in abutment with the facial area, generally preventing all visual impressions incoming at those facial areas.

5. Device according to any of the preceding claims, further comprising a third limiter (30) comprising third surface areas (32) capable of limiting impressions in front of the eye area of the user.

6. Device according to claim 7, wherein the third surface areas (32) of the third limiter (30) extend from generally each pupil of the user and outwards.

7. Device according to claim 7 or 8, wherein the third surface areas (32) of the third limiter (30) is arranged to allow sight visibility in downwards directions.

8. Device according to claim 8 or 9, wherein the third surface areas (32) of the third limiter (30) is arranged as a part of fourth surface areas (34) in front of the eye area.

9. Device according to claim 10, wherein the remainder of the fourth surface areas (34) are transparent.

10. Device according to claim 11, wherein the remainder of the fourth surface areas (34) comprise sight correcting features.

11. Device according to claim 11 or 12, wherein the remainder of the fourth surface areas comprise light spectra limiting features.

12. Device according to any of the preceding claims 7 to 13, wherein the third surface areas (32) of the third limiter (30) are arranged generally planar and that the surface areas in front of each eye preferably are generally parallel to each other.

13. Device according to any of the preceding claims 10 to 13, wherein the fourth surface areas (34) are arranged generally planar and that the surface areas in front of each eye preferably are generally parallel to each other.

14. Device according to any of the preceding claims, wherein the carrier structure comprises spectacles, providing carrier support on the nose and the ears of a user.

15. Device according to any of the claims 1 to 15, wherein the carrier structure is arranged to be attached to spectacles worn by a user.
